# EUROPEAN PATENT APPLICATION

(11) **EP 4 810 078 A1**
(43) Date of publication of application: **23.09.2026**
(21) Application number: 26153685.8
(22) Date of filing: 23.01.2026
(51) Int. Cl.: A61M 5/14, A61M 5/145, A61M 5/168, A61M 39/28

(54) **MEDICAL FLUID FLOW DEVICES**

(30) Priority: 20.03.2025 US 202519085978
(71) Applicant: CareFusion 303, Inc., San Diego, CA 92130 (US)
(72) Inventor: GRIFFIN, John-Paul, San Diego , CA 92130 (US); OOI, Chun Keat, San Diego , CA 92130 (US); GALVIN, Michael A, San Diego , CA 92130 (US)
(74) Representative: Zenz Patentanwälte Partnerschaft mbB

(57) **Abstract**

Medical fluid flow devices are provided with a reusable control portion that includes a chassis, a fluid flow controller coupled to the chassis, a fluid flow valve coupled to the fluid flow controller, an actuator coupled to the chassis and a housing cover coupled to the chassis. A disposable fluid output portion includes a housing, an internal fluid pathway, a fluid input port, a fluid output port fluidly coupled to the fluid pathway, a fluid reservoir disposed within the housing and fluidly coupled to the fluid pathway, a plunger moveably disposed within the fluid reservoir and a valve cavity disposed into the housing. When the disposable fluid output portion is coupled to the reusable control portion, the fluid flow valve is disposed within the valve cavity and the plunger is disposed within the fluid reservoir. Fluid flow sets and methods of operating medical fluid flow devices are also provided.

## Description

### CROSS-REFERENCES TO RELATED APPLICATIONS

The present application claim priority to U.S. Patent Application No. 19/085,978, entitled "MEDICAL FLUID FLOW DEVICES," filed on March 20, 2025, the entirety of which is incorporated herein by reference.

### BACKGROUND

A patient, especially in an intensive care unit (ICU), may require multiple infusion pumps and intravenous (IV) fluid lines (e.g. 10 or more lines). However, the patient may only have a few patient ports, such as one peripheral catheter and one central catheter. Thus, multiple lines/pumps have to infuse into the same patient port. Typically, medical fluid flow manifolds are used in the medical field for controlling fluid flow to a patient, the manifold having multiple lines from pumps connected to a series of 3-way stopcocks. A stopcock mechanism may be easy to turn but is not necessarily intuitive to use, such as which direction will result in restricting or opening the fluid flow. Also, a typical line labeling and tubing guide process involves a manual labeling/numbering process upstream and downstream of the pump mechanisms. In addition, a separate syringe pre-filled with saline is typically used for manual flushing of lines and manifold ports.

Accordingly, a typical clinical workflow when switching between pumps with different drugs has potential risks and issues. A clinician has to manually trace or determine which IV line of many belongs to an infusion that is ending, which results in infusion line confusion. Once the line is determined, the clinician must manually close the 3-way stopcock on the manifold for that line where the resulting line manipulation may disturb the patient port. The clinician then has to manually prepare a pre-filled syringe or fill an empty syringe with saline and manually flush the line with saline, which is time consuming with additional cost and the resulting handling may result in increased infection risks to the patient. Then, the clinician must determine which IV line belongs to the next infusion that is to be started and manually open the 3-way stopcock on the manifold for that infusion, again resulting in infusion line confusion and additional line manipulation that may disturb the patient port.

### SUMMARY

The present disclosure provides medical fluid flow devices and in particular automated medical fluid flow devices with reusable and disposable components.

In one or more embodiments, a medical fluid flow device comprises a reusable control portion and a disposable fluid output portion. The reusable control portion comprises a chassis, a fluid flow controller coupled to the chassis, a fluid flow valve coupled to the fluid flow controller, an actuator coupled to the chassis and a housing cover coupled to the chassis. The disposable fluid output portion comprises a housing, an internal fluid pathway, a fluid input port, a fluid output port fluidly coupled to the fluid pathway, a fluid reservoir disposed within the housing and fluidly coupled to the fluid pathway, a plunger moveably disposed within the fluid reservoir and a valve cavity disposed into the housing. When the disposable fluid output portion is coupled to the reusable control portion, the fluid flow valve is disposed within the valve cavity and the plunger is configured to be engaged by the actuator, wherein the fluid flow valve is configured to fluidly couple the fluid input port to the fluid pathway when the fluid flow valve is in an open position and the fluid flow valve is configured to occlude a fluid coupling between the fluid input port and the fluid pathway when the fluid flow valve is in a closed position.

In one or more embodiments, a fluid flow set, comprises a disposable fluid output portion for a medical fluid flow device, the disposable fluid output portion comprising a housing, an internal fluid pathway, a fluid input port fluidly coupled to the fluid pathway, a fluid output port fluidly coupled to the fluid pathway, a fluid reservoir disposed within the housing and fluidly coupled to the fluid pathway, a plunger moveably disposed within the fluid reservoir and a valve cavity disposed into the housing. The fluid flow set also includes a fluid inlet tube coupled to the fluid input port and a fluid outlet tube coupled to the fluid output port, wherein the disposable fluid output portion is configured to be removably coupled to a reusable control portion of the medical fluid flow device.

In one or more embodiments, a method of operating a medical fluid flow device comprises coupling a fluid flow set to a reusable control portion of a medical fluid flow device, the fluid flow set comprising: a disposable fluid output portion comprising a housing, an internal fluid pathway, a fluid input port fluidly coupled to the fluid pathway, a fluid output port fluidly coupled to the fluid pathway, a fluid reservoir disposed within the housing and fluidly coupled to the fluid pathway, a plunger moveably disposed within the fluid reservoir and a valve cavity disposed into the housing; a fluid inlet tube having a first end coupled to the fluid input port; and a fluid outlet tube having a first end coupled to the fluid output port. The method also comprises: coupling a second end of the fluid inlet tube to an intravenous pump; coupling a second end of the fluid outlet tube to a medication delivery port; entering, via a touchscreen of the reusable control portion, at least one of a pump number and a drug name corresponding to the fluid inlet tube; pressing an on button on the touchscreen to run an infusion of the drug from the intravenous pump to the medication delivery port; opening, by a fluid flow controller of the reusable control portion, a valve coupled to the fluid flow controller to fluidly connect the fluid inlet tube with the fluid pathway in the disposable fluid output portion; pressing an off button on the touchscreen to end the infusion of the drug from the intravenous pump to the medication delivery port; closing, by the fluid flow controller of the reusable control portion, the valve to close the fluid connection between the fluid inlet tube and the fluid pathway in the disposable fluid output portion; automatically activating an actuator of the reusable control portion to push the plunger into the fluid reservoir in the disposable fluid output portion; and forcing, by the plunger, saline from the reservoir to flush through the fluid pathway and out to the fluid outlet tube.

Additional features and advantages of the disclosure will be set forth in the description below and, in part, will be apparent from the description or may be learned by practice of the disclosure. The objectives and other advantages of the disclosure will be realized and attained by the structure particularly pointed out in the written description and claims hereof as well as the appended drawings.

It is to be understood that both the foregoing general description and the following detailed description are exemplary and explanatory and are intended to provide further explanation of the disclosure as claimed.

### BRIEF DESCRIPTION OF THE DRAWINGS

The accompanying drawings, which are included to provide further understanding of the disclosure and are incorporated in and constitute a part of this specification, illustrate embodiments of the disclosure and together with the description serve to explain the principles of the disclosure.
FIG. 1 depicts a typical IV pump and fluid line setup for a patient.
FIG. 2 is a top view of a typical fluid flow manifold with stopcocks.
FIG. 3 depicts several typical IV pump and fluid line setups.
FIG. 4 is a perspective view of an automated medical fluid flow device, according to aspects of the disclosure.
FIG. 5 is a perspective view of an example of the automated medical fluid flow device of FIG. 4 with a housing cover removed, according to aspects of the disclosure.
FIG. 6 is a perspective view of an example of the automated medical fluid flow device of FIG. 4 with a housing cover removed, according to aspects of the disclosure.
FIG. 7 is a rear view of the automated medical fluid flow device of FIG. 6 with the IV lines removed, according to some aspects of the disclosure.
FIG. 8 is a bottom view of the automated medical fluid flow device of FIG. 7, according to some aspects of the disclosure.
FIG. 9 is a top view of the automated medical fluid flow device of FIG. 7, according to some aspects of the disclosure.
FIG. 10 is a front view of a reusable portion of an automated medical fluid flow device, according to aspects of the disclosure.
FIG. 11 is a perspective view of a disposable portion of an automated medical fluid flow device, according to aspects of the disclosure.
FIG. 12 is a perspective view of a disposable portion of an automated medical fluid flow device, according to aspects of the disclosure.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions are provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

Fluid flow sets (e.g., IV sets) may be formed from any combination of fluid flow components and tubing. Typically, the fluid flow components and tubing are fixedly connected together to form a disposable fluid flow set that is used once and then discarded. The fluid flow components and tubing may be formed from any suitable material (e.g., plastic, silicone, rubber).

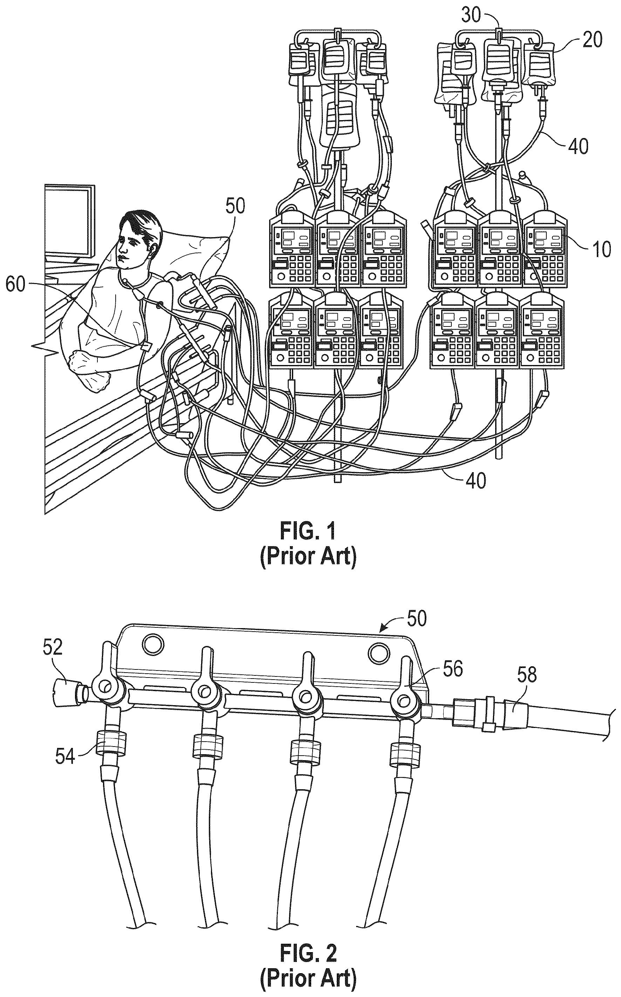

A typical IV pump and fluid line configuration is shown in FIG. 1. IV pumps 10 and fluid containers 20 are hanging on IV poles 30. IV lines 40 run from the fluid containers 20 to the IV pumps 10 and from the IV pumps 10 to fluid flow manifolds 50 and/or to a patient port 60.

A typical fluid flow manifold 50 is shown in FIG. 2 as a four port fluid manifold having a saline inlet port 52, four drug inlet ports 54 controlled by stopcocks 56 and an outlet port 58.

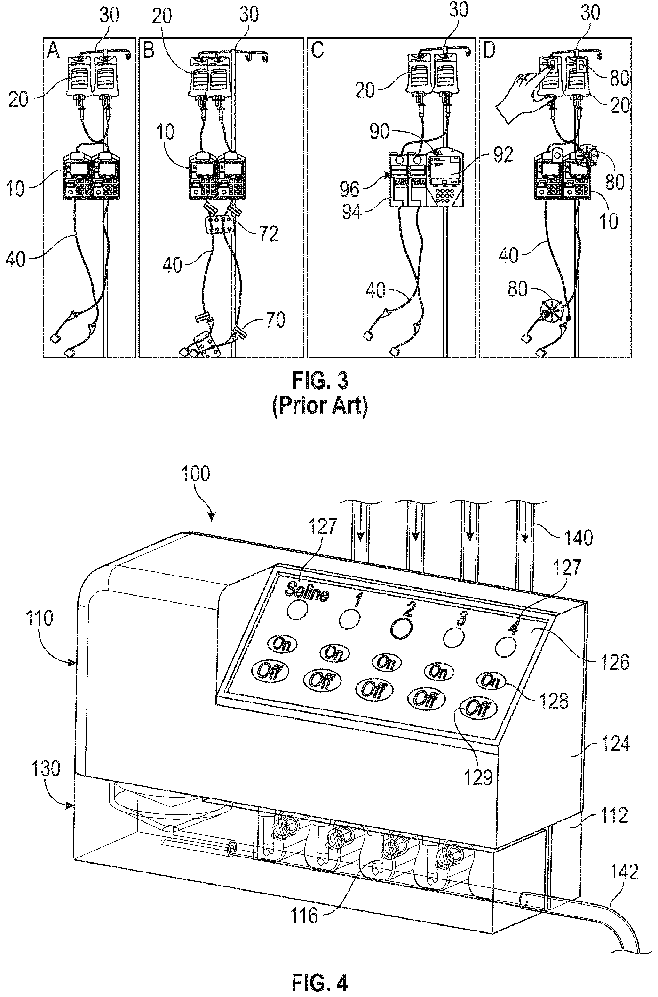

Some typical solutions to the issues and risks of IV systems discussed above are shown in FIG. 3. FIG. 3A shows a typical two IV pump 10 setup having two fluid containers 20 and two unlabeled IV lines 40 configured to go from the IV pumps 10 to a patient. FIG. 3B shows the IV system of FIG. 3A with the addition of line labels 70 and tubing guides 72 for identifying and straightening IV lines 40 related to respective IV pumps 10. FIG. 3D shows the IV system of FIG. 3A with the addition of lighting transmitter/receiver pairs 80 for identifying IV lines 40 with respective IV pumps 10 and fluid containers 20. FIG. 3C shows a typical IV smart pump system having a pump control unit 90 with a central display 92 and two pumps 94 each having a scrolling marquee 96, where each of the central display 92 and the scrolling marquee 96 display a drug name and dose associated with the pump 94. However, applying any or all of these solutions shown in FIGS. 3B, 3C and 3D to the system shown in FIG. 1 still leaves some issues and risks discussed above.

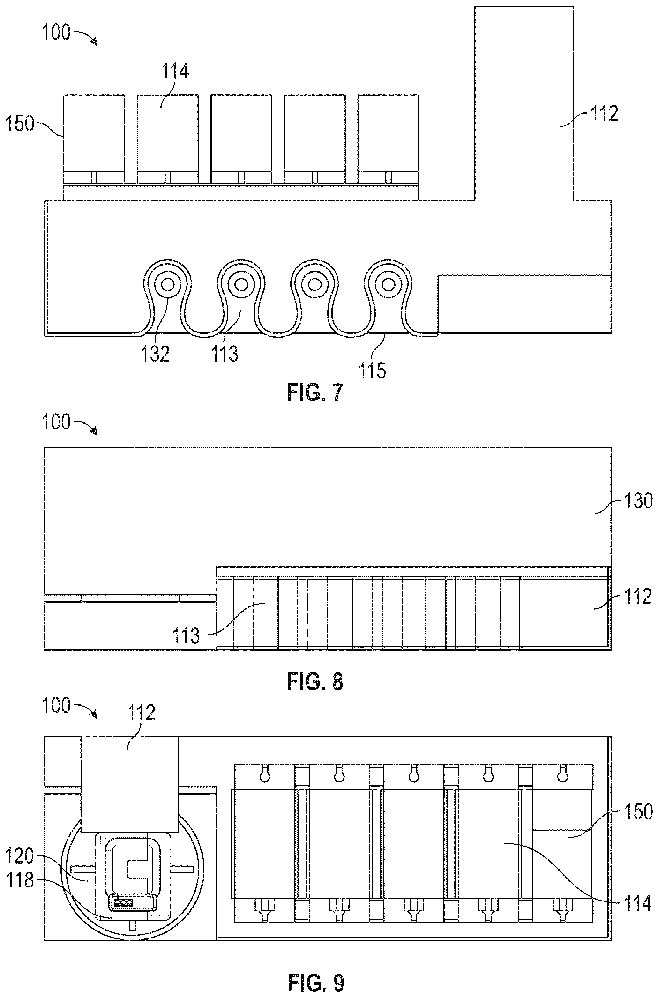

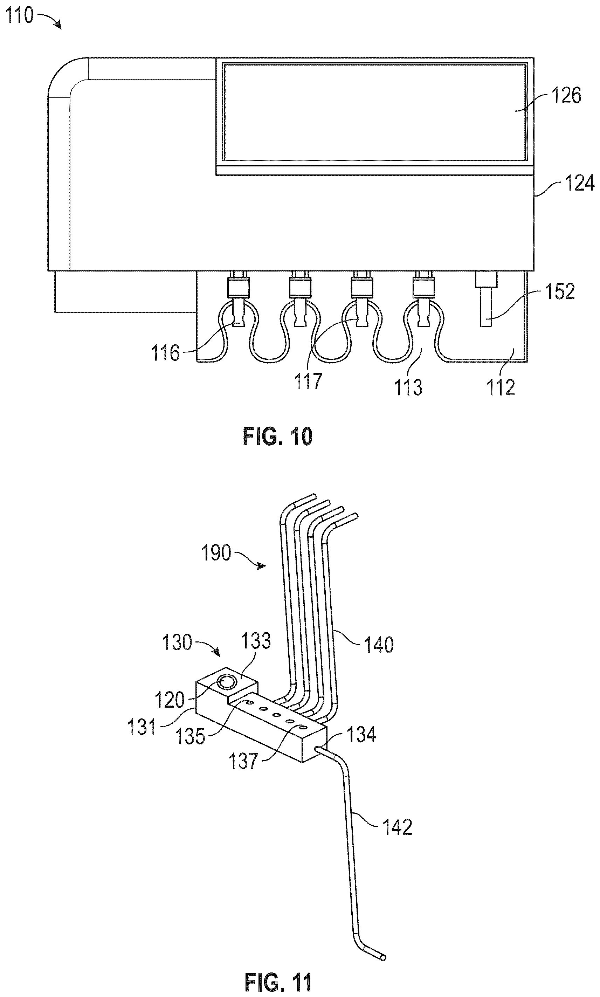

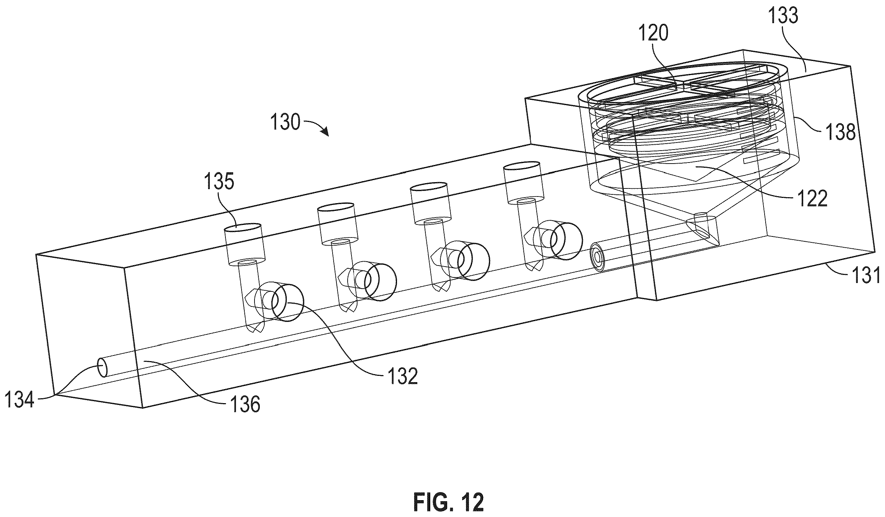

FIGS. 4-12 illustrate an automated medical fluid flow device shown as fluid flow device 100, according to some aspects of the disclosure. The fluid flow device 100 has a reusable fluid control portion 110 and a disposable fluid output portion 130 removably coupled to the control portion 110. The control portion includes four fluid flow controllers 114 (e.g., micro servo, switch valve) coupled to a main chassis 112. The fluid flow controllers 114 may be removably coupled to the chassis 112 in any suitable way (e.g., screws, snaps, clips). Each fluid flow controller 114 is coupled to a valve 116 for controlling fluid flow from a fluid input line 140 out through a fluid output line 142.

The fluid flow device 100 as shown has four fluid input ports 132 and one fluid output port 134, though any desired number of fluid input ports 132 and fluid output ports 134 are contemplated. The input ports 132 are each accessible via line cavities 113 in the chassis 112. The line cavities 113 may be essentially three sided with an open portion 115 at the bottom of the chassis 112. In aspects of the disclosure, the line cavities 113 may be holes in the rear of the chassis 112 or any other suitable opening, for example.

The reusable fluid control portion 110 also includes an actuator 118 (e.g., linear actuator) coupled to the chassis 112, the actuator 118 configured to engage with the disposable fluid output portion 130 when the disposable fluid output portion 130 and the reusable fluid control portion 110 are coupled together (e.g., snapped together, connected). A housing cover 124 is coupled to the chassis 112. A display 126 (e.g., touchscreen, membrane) may be coupled to or integrated with the housing cover 124. For example, as shown in FIG. 4, the display 126 has indicators 127 for a flushing medium (e.g., saline) and fluid input lines with on buttons 128 and off buttons 129 for each. The display 126 may provide a visual status of which of the flushing medium flushing process and medication lines are currently running or not. The on buttons 128 and off buttons 129 may also receive touch input to start or stop any of the flushing process or medication fluid flow from one or more of the input lines 140.

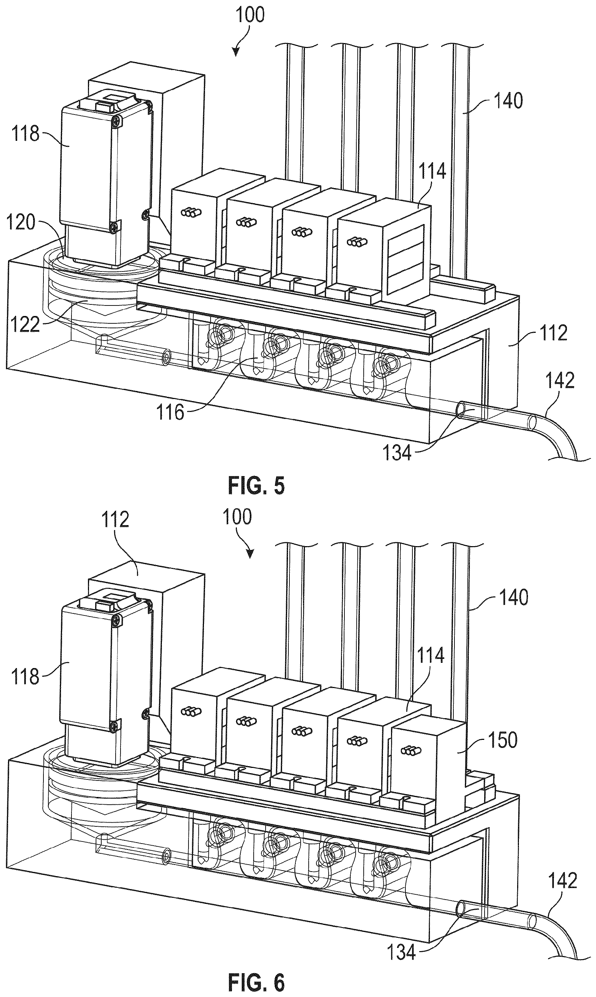

As shown in FIG. 6, a sensor 150 assembly may be coupled to the chassis 112 in any suitable manner, such as the removable couplings discussed above for the fluid flow controller 114. The sensor assembly 150 may be any desired sensor system, such as a pressure sensor, an optical sensor or any other suitable sensor. The sensor assembly 150 may monitor or measure any desirable attribute, such as flow rate measurement, air-in-line detection, in-line pressure monitoring and/or provide configurable alarm levels, for example. Here, having one common measurement point from the sensor assembly 150 instead of sensors on each individual pump 10 and IV set line 40 may decrease cost and provide the best possible accuracy.

In aspects of the disclosure, the sensor assembly 150 may provide monitoring for air-in-line and/or elimination of air-in-line. In aspects of the disclosure, the sensor assembly 150 may provide flow rate measurement and/or feedback to correct pump error. In aspects of the disclosure, the sensor assembly 150 may provide measurement of downstream line pressure. In aspects of the disclosure, the sensor assembly 150 may function as an alarm system (e.g., excessive air/pressure, over/under infusion, free flow).

There are several advantages of the fluid flow device 100 having a sensor assembly 150. For example, sensing technologies come at a cost, especially since higher-accuracy options are typically disposable (e.g., in-line measurement). Having one in-line measurement for multiple fluidic inputs may increase cost efficiency while achieving the best possible accuracy. Similarly, the sensing assembly 150 may provide the option to have an alarm system, again one system for multiple input lines. Accurate sensing for all lines at reasonable cost may provide increased patient safety and less nuisance alarms. In addition, the sensing assembly 150 may provide flow rate correction at the fluid flow device 100 level.

The disposable fluid output portion 130 has a housing 131 with the fluid input ports 132 and the fluid output port 134 coupled to a fluid pathway 136. The fluid input ports 132 are each configured to receive one of the fluid input lines 140 (e.g., flexible IV tubing) and the fluid outlet port 134 is configured to receive the fluid output line 142, which also may be IV tubing. Thus, each fluid input port 132/fluid input line 140 coupling and each fluid outlet port 134/fluid output line 142 coupling are fluidly or fluidically coupled.

A fluid reservoir 138 is disposed within the housing 131 and is also coupled to the fluid pathway 136. A plunger 120 and a rubber tip 122 coupled to the plunger 120 are moveably disposed or contained within the fluid reservoir 138, the plunger 120 configured to couple with or be engaged by the actuator 118 of the reusable portion 110. In aspects of the disclosure, the fluid reservoir 138 may be sized and shaped to contain any suitable actuation member for forcing some or all of the fluid within the fluid reservoir 138 out through the fluid pathway 136.

The fluid reservoir 138 may be pre-filled with a suitable flushing medium (e.g., saline, any other infusate) in order to provide fluid flushes through the fluid pathway 136, for example. Here, the plunger 120 seals the flushing medium within the fluid reservoir 138 so that the disposable fluid output portion 130 may be shipped, stored and otherwise handled without leakage of the flushing medium. When the disposable fluid output portion 130 is coupled to the reusable fluid control portion 110, the plunger 120 may be pushed into or further into the fluid reservoir 138 by the actuator 118 so that the rubber tip 122 forces an amount of flushing medium out of the fluid reservoir 138, through the fluid pathway 136 and out of the fluid outlet port 134 to flush any remaining medication fluid from one or more input ports 132.

In aspects of the disclosure, the plunger 120 may be initially positioned so that a portion of the plunger 120 extends out past a surface 133 of the housing 131, an outer surface of the plunger 120 is flush with the surface 133 of the housing 131, or an outer surface of the plunger 120 is recessed below the surface 133 of the housing 131. In aspects of the disclosure, the plunger 120 may be initially positioned within the fluid reservoir 138 where initial activation of the actuator 118 moves the plunger 120 further into the fluid reservoir 138. In aspects of the disclosure, the plunger 120 may be initially positioned adjacent the fluid reservoir 138 where initial activation of the actuator 118 moves the plunger 120 into the fluid reservoir 138 and further activation of the actuator 118 moves the plunger 120 further into the fluid reservoir 138.

Valve cavities 135 are also disposed in the housing 131, each valve cavity 135 being configured to receive one of the valves 116 of the reusable portion 110. Here, the valve 116 may be turned by the corresponding flow controller 114 to open or close a fluid connection between the corresponding fluid input port 132 and the fluid pathway 136, such as turning the valve 116 by 90 degrees for example. For example, the valve 116 may have a fluid channel 117 that may be aligned with the fluid input port 132 in the open position and not aligned with the fluid input port 132 in the closed position. In aspects of the disclosure, the valve 116 may be turned less than 90 degrees to provide a partial fluid alignment of the fluid channel 117 between the fluid input port 132 and the fluid pathway 136, thus providing a form of flow control to the fluid entering the fluid input port 132 from the fluid input line 140.

In aspects of the disclosure, a sensor cavity 137 may be disposed in the housing 131, the sensor cavity 137 configured to receive a sensor member 152 of the sensor assembly 150. Here, a part of the sensor member 152 may be in contact with the fluid pathway 136 (e.g., pressure sensor to measure fluid pressure) and/or a part of the sensor member 152 may be adjacent the fluid pathway 136 in a non-contact manner (e.g., optical transmitter/receiver pair to measure gas bubbles in the fluid). Any suitable sensor assembly 150 may be used to obtain the desired measurements.

A disposable IV set 190 may include any combination of the disposable fluid output portion 130, the fluid input lines 140 and the fluid output line 142, either as separate components or as a pre-assembled set. In aspects of the disclosure, the disposable fluid output portion 130 of the IV set 190 may be coupled (e.g., snapped on) to the reusable portion 110 of the fluid flow device 100 to provide a pre-filled reservoir 138 of flushing medium and fresh input/output lines 140, 142. In aspects of the disclosure, only a disposable fluid output portion 130 of the fluid flow device 100 may be coupled (e.g., snapped on) to the reusable portion 110 of the fluid flow device 100 to provide a pre-filled reservoir 138 of flushing medium, while existing input/output lines 140, 142 are coupled to the new disposable fluid output portion 130. Here, the existing input/output lines 140, 142 may have been detached from a previous disposable fluid output portion 130 where the flushing medium reservoir 138 is empty and/or for lifecycle time management, for example. In aspects of the disclosure, any combination of the disposable fluid output portion 130, the fluid input lines 140 and the fluid output line 142 may be exchanged separately or together.

The disposable fluid output portion 130 may be manufactured as a complete assembly, including any or all of the inlet ports 132, the outlet port 134, the fluid reservoir 138, the valve cavities 135 and the sensor cavity 137, any of which may further be protected with a protective cap or cover that may be removed for use. Thus, each fluid entry or exit point of the main fluid pathway 136 may be protected from contamination until its use. Here, the fluid reservoir 138 may be pre-filled and sealed during manufacturing. In aspects of the disclosure, the fluid reservoir 138 may be fillable with a flushing medium in the field (e.g., by syringe).

The inlet ports 132 and the outlet port 134 may include any suitable connector (e.g., male luer connector, female luer connector, proprietary connector) and the corresponding fluid input lines 140 and fluid output line 142 may include any suitable corresponding connector (e.g., female luer connector, male luer connector, proprietary connector). For example, the outlet port 134 may have a male luer connector and the fluid output line 142 may have a corresponding female luer connector that mates with the male luer connector of the outlet port 134. As another example, each inlet port 132 and each corresponding fluid input line 140 may have proprietary connectors that mate with each other, thus allowing only the appropriate disposable fluid output portion 130 to be coupled to or connected with the reusable fluid control portion 110 and/or only allowing the appropriate fluid input line 140 to be connected to a particular inlet port 132 of the disposable fluid output portion 130.

The fluid flow device 100 is configured to be intuitive to use, to be safe to use with any pump systems and/or IV sets and to provide flow control for each drug port 132. The fluid flow device 100 may be configured for use with liquid fluid flow, according to some aspects of the disclosure. The fluid flow device 100 may be configured for use with gaseous fluid flow, according to some aspects of the disclosure.

In use, a workflow process of the fluid flow device 100 may include an initial set up to connect the disposable portion 130 to the reusable portion 110, connect each IV line 140 coming from a pump (e.g., pump 10, 94) to a fluid input port 132, program the corresponding pump number and/or drug name into the fluid flow device 100 and connect a fluid output line 142 to the fluid output port 134.

The fluid flow device 100 provides many improvements to the typical clinical workflow of IV infusion into a patient. For example, the fluid flow device 100 eliminates or minimizes infusion line confusion during set up and during the infusion process. Also, after a first infusion from one input line 140 is finished, the clinician merely presses the corresponding off button 129 on the display 126 to close the corresponding valve 116, resulting in no line manipulation to disturb the patient. After each infusion, the fluid flow device 100 may automatically flush the fluid pathway 136 with flushing medium from the reservoir 138, thus eliminating any additional time/effort by the clinician and eliminating the need for a clinician to manually use a flushing medium filled syringe.

To start the next infusion (e.g., second infusion), the clinician presses the on button 128 on the display 126 for the appropriate IV line 140 to open the corresponding valve 116, again resulting in no line manipulation to disturb the patient while reducing process steps and improving clinical efficiency. The infusion start and stop processes may be repeated for any remaining IV lines 140. In aspects of the disclosure, multiple IV lines 140 may be opened and closed for a combination infusion cycle. For example, a first drug from a first IV line 140 and a second drug from a second IV line 140 may both be infused through the fluid flow device 100 at the same time or for part of the same time, with a flushing medium flush cycle occurring after one or both drug infusions are completed.

At any time during setup or infusion to a patient, the disposable portion 130 may be disconnected from the reusable portion 110. For example, once a predetermined series of infusions have occurred, the flushing medium reservoir 138 is empty and/or the lifetime of the IV set is expired may be causes for removing the disposable portion 130 and/or replacing the used disposable portion 130 with a fresh disposable portion 130.

In one or more embodiments, a medical fluid flow device comprises a reusable control portion and a disposable fluid output portion. The reusable control portion comprises a chassis, a fluid flow controller coupled to the chassis, a fluid flow valve coupled to the fluid flow controller, an actuator coupled to the chassis and a housing cover coupled to the chassis. The disposable fluid output portion comprises a housing, an internal fluid pathway, a fluid input port, a fluid output port fluidly coupled to the fluid pathway, a fluid reservoir disposed within the housing and fluidly coupled to the fluid pathway, a plunger moveably disposed within the fluid reservoir and a valve cavity disposed into the housing. When the disposable fluid output portion is coupled to the reusable control portion, the fluid flow valve is disposed within the valve cavity and the plunger is configured to be engaged by the actuator, wherein the fluid flow valve is configured to fluidly couple the fluid input port to the fluid pathway when the fluid flow valve is in an open position and the fluid flow valve is configured to occlude a fluid coupling between the fluid input port and the fluid pathway when the fluid flow valve is in a closed position.

In aspects of the disclosure, the reusable control portion comprises a plurality of fluid flow controllers and a plurality of fluid flow valves each coupled to a corresponding fluid flow controller, and wherein the disposable fluid output portion comprises matching plurality of valve cavities and corresponding fluid input ports. In aspects of the disclosure, each of the fluid input ports and the fluid output port is configured to receive and fluidly couple with a separate intravenous tube. In aspects of the disclosure, the fluid reservoir contains a flushing medium and the actuator is configured to move the plunger further into the fluid reservoir to flush the flushing medium into the fluid pathway. In aspects of the disclosure, the plunger includes a rubber tip that comprises a contact surface of the plunger with the flushing medium.

In aspects of the disclosure, the chassis comprises a line cavity and wherein the fluid input port receives and is fluidly coupled to an intravenous tube that passes through the line cavity. In aspects of the disclosure, the line cavity has an open portion facing a bottom surface of the chassis, and wherein the line cavity is configured to receive the intravenous tube that is already coupled to the fluid input port. In aspects of the disclosure, a control display is coupled to the housing cover of the reusable control portion. In aspects of the disclosure, the control display comprises a flushing medium indicator and an input line indicator, each with corresponding on and off control buttons.

In aspects of the disclosure, a sensor assembly is coupled to the chassis. In aspects of the disclosure, the sensor assembly is configured to provide one of flow rate measurement, air-in-line detection and in-line pressure monitoring. In aspects of the disclosure, the sensor assembly is configured to provide one of an excessive air alarm, as excessive pressure alarm, an over infusion alarm, an under infusion alarm and a free flow alarm. In aspects of the disclosure, the housing of the disposable fluid output portion comprises a sensor cavity configured to receive a sensor member of the sensor assembly when the disposable fluid output portion is coupled to the reusable control portion.

In one or more embodiments, a fluid flow set, comprises a disposable fluid output portion for a medical fluid flow device, the disposable fluid output portion comprising a housing, an internal fluid pathway, a fluid input port fluidly coupled to the fluid pathway, a fluid output port fluidly coupled to the fluid pathway, a fluid reservoir disposed within the housing and fluidly coupled to the fluid pathway, a plunger moveably disposed within the fluid reservoir and a valve cavity disposed into the housing. The fluid flow set also includes a fluid inlet tube coupled to the fluid input port and a fluid outlet tube coupled to the fluid output port, wherein the disposable fluid output portion is configured to be removably coupled to a reusable control portion of the medical fluid flow device.

In aspects of the disclosure, the fluid reservoir is pre-filled with a flushing medium. In aspects of the disclosure, the valve cavity is configured to receive a fluid flow valve of the reusable control portion and the plunger is configured to couple with the actuator the reusable control portion when the disposable fluid output portion is coupled to the reusable control portion of the medical fluid flow device.

In aspects of the disclosure, a sensor cavity is disposed into the housing and is configured to receive a sensor member of a sensor assembly of the reusable control portion when the disposable fluid output portion is coupled to the reusable control portion of the medical fluid flow device. In aspects of the disclosure, the fluid inlet tube coupled to the fluid input port is configured to pass through an open side of a line cavity disposed in a chassis of the reusable control portion when the disposable fluid output portion is coupled to the reusable control portion of the medical fluid flow device.

In one or more embodiments, a method of operating a medical fluid flow device comprises coupling a fluid flow set to a reusable control portion of a medical fluid flow device, the fluid flow set comprising: a disposable fluid output portion comprising a housing, an internal fluid pathway, a fluid input port fluidly coupled to the fluid pathway, a fluid output port fluidly coupled to the fluid pathway, a fluid reservoir disposed within the housing and fluidly coupled to the fluid pathway, a plunger moveably disposed within the fluid reservoir and a valve cavity disposed into the housing; a fluid inlet tube having a first end coupled to the fluid input port; and a fluid outlet tube having a first end coupled to the fluid output port. The method also comprises: coupling a second end of the fluid inlet tube to an intravenous pump; coupling a second end of the fluid outlet tube to a medication delivery port; entering, via a touchscreen of the reusable control portion, at least one of a pump number and a drug name corresponding to the fluid inlet tube; pressing an on button on the touchscreen to run an infusion of the drug from the intravenous pump to the medication delivery port; opening, by a fluid flow controller of the reusable control portion, a valve coupled to the fluid flow controller to fluidly connect the fluid inlet tube with the fluid pathway in the disposable fluid output portion; pressing an off button on the touchscreen to end the infusion of the drug from the intravenous pump to the medication delivery port; closing, by the fluid flow controller of the reusable control portion, the valve to close the fluid connection between the fluid inlet tube and the fluid pathway in the disposable fluid output portion; automatically activating an actuator of the reusable control portion to push the plunger into the fluid reservoir in the disposable fluid output portion; and forcing, by the plunger, saline from the reservoir to flush through the fluid pathway and out to the fluid outlet tube.

In aspects of the disclosure, the fluid flow set is a first fluid flow set and the method further comprises replacing the first fluid flow set by: disconnecting the disposable fluid output portion of the first fluid flow set from the reusable control portion of the medical fluid flow device; disconnecting the fluid inlet tube of the first fluid flow set from the intravenous pump; disconnecting the fluid outlet tube of the first fluid flow set from the medication delivery port; connecting a disposable fluid output portion of a second fluid flow set having a reservoir pre-filled with a flushing medium to the reusable control portion of the medical fluid flow device; connecting a fluid inlet tube of the second fluid flow set to the intravenous pump; and connecting a fluid outlet tube of the second fluid flow set to the medication delivery port.

It is understood that any specific order or hierarchy of blocks in the methods of processes disclosed is an illustration of example approaches. Based upon design or implementation preferences, it is understood that the specific order or hierarchy of blocks in the processes may be rearranged, or that all illustrated blocks be performed. In some implementations, any of the blocks may be performed simultaneously.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

As used herein, the phrase "at least one of" preceding a series of items, with the term "or" to separate any of the items, modifies the list as a whole, rather than each item of the list. The phrase "at least one of" does not require selection of at least one item; rather, the phrase allows a meaning that includes at least one of any one of the items, and/or at least one of any combination of the items, and/or at least one of each of the items. By way of example, the phrase "at least one of A, B, or C" may refer to: only A, only B, or only C; or any combination of A, B, and C.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

It is understood that the specific order or hierarchy of steps, operations or processes disclosed is an illustration of exemplary approaches. Based upon design preferences, it is understood that the specific order or hierarchy of steps, operations or processes may be rearranged. Some of the steps, operations or processes may be performed simultaneously. Some or all of the steps, operations, or processes may be performed automatically, without the intervention of a user. The accompanying method claims, if any, present elements of the various steps, operations or processes in a sample order, and are not meant to be limited to the specific order or hierarchy presented.

All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed under the provisions of 35 U.S.C. §112 (f) unless the element is expressly recited using the phrase "means for" or, in the case of a method claim, the element is recited using the phrase "step for." Furthermore, to the extent that the term "include," "have," or the like is used, such term is intended to be inclusive in a manner similar to the term "comprise" as "comprise" is interpreted when employed as a transitional word in a claim.

The Title, Background, Summary, Brief Description of the Drawings and Abstract of the disclosure are hereby incorporated into the disclosure and are provided as illustrative examples of the disclosure, not as restrictive descriptions. It is submitted with the understanding that they will not be used to limit the scope or meaning of the claims. In addition, in the Detailed Description, it can be seen that the description provides illustrative examples and the various features are grouped together in various embodiments for the purpose of streamlining the disclosure. This method of disclosure is not to be interpreted as reflecting an intention that the claimed subject matter requires more features than are expressly recited in each claim. Rather, as the following claims reflect, inventive subject matter lies in less than all features of a single disclosed configuration or operation. The following claims are hereby incorporated into the Detailed Description, with each claim standing on its own as a separately claimed subject matter.

The claims are not intended to be limited to the aspects described herein, but are to be accorded the full scope consistent with the language claims and to encompass all legal equivalents. Notwithstanding, none of the claims are intended to embrace subject matter that fails to satisfy the requirement of 35 U.S.C. § 101, 102, or 103, nor should they be interpreted in such a way.

## Claims

1. A medical fluid flow device, comprising:
a reusable control portion, comprising:
a chassis;
a fluid flow controller coupled to the chassis;
a fluid flow valve coupled to the fluid flow controller;
an actuator coupled to the chassis; and
a housing cover coupled to the chassis; and
a disposable fluid output portion, comprising:
a housing;
an internal fluid pathway;
a fluid input port;
a fluid output port fluidly coupled to the fluid pathway;
a fluid reservoir disposed within the housing and fluidly coupled to the fluid pathway;
a plunger moveably disposed within the fluid reservoir; and
a valve cavity disposed into the housing,
wherein when the disposable fluid output portion is coupled to the reusable control portion, the fluid flow valve is disposed within the valve cavity and the plunger is configured to be engaged by the actuator,
wherein the fluid flow valve is configured to fluidly couple the fluid input port to the fluid pathway when the fluid flow valve is in an open position and the fluid flow valve is configured to occlude a fluid coupling between the fluid input port and the fluid pathway when the fluid flow valve is in a closed position.

2. The medical fluid flow device of claim 1, wherein the reusable control portion comprises a plurality of fluid flow controllers and a plurality of fluid flow valves each coupled to a corresponding fluid flow controller, and wherein the disposable fluid output portion comprises matching plurality of valve cavities and corresponding fluid input ports.

3. The medical fluid flow device of claim 2, wherein each of the fluid input ports and the fluid output port is configured to receive and fluidly couple with a separate intravenous tube.

4. The medical fluid flow device of claim 1, wherein the fluid reservoir contains a flushing medium and the actuator is configured to move the plunger further into the fluid reservoir to flush the flushing medium into the fluid pathway.

5. The medical fluid flow device of claim 4, wherein the plunger includes a rubber tip that comprises a contact surface of the plunger with the flushing medium.

6. The medical fluid flow device of claim 1, wherein the chassis comprises a line cavity and wherein the fluid input port receives and is fluidly coupled to an intravenous tube that passes through the line cavity.

7. The medical fluid flow device of claim 6, wherein the line cavity has an open portion facing a bottom surface of the chassis, and wherein the line cavity is configured to receive the intravenous tube that is already coupled to the fluid input port.

8. The medical fluid flow device of claim 1, further comprising a control display coupled to the housing cover of the reusable control portion.

9. The medical fluid flow device of claim 8, wherein the control display comprises a flushing medium indicator and an input line indicator, each with corresponding on and off control buttons.

10. The medical fluid flow device of claim 1, further comprising a sensor assembly coupled to the chassis.

11. The medical fluid flow device of claim 10, wherein the sensor assembly is configured to provide one of flow rate measurement, air-in-line detection and in-line pressure monitoring.

12. The medical fluid flow device of claim 10, wherein the sensor assembly is configured to provide one of an excessive air alarm, as excessive pressure alarm, an over infusion alarm, an under infusion alarm and a free flow alarm.

13. The medical fluid flow device of claim 10, wherein the housing of the disposable fluid output portion comprises a sensor cavity configured to receive a sensor member of the sensor assembly when the disposable fluid output portion is coupled to the reusable control portion.

14. A fluid flow set, comprising:
a disposable fluid output portion for a medical fluid flow device, the disposable fluid output portion comprising:
a housing;
an internal fluid pathway;
a fluid input port fluidly coupled to the fluid pathway;
a fluid output port fluidly coupled to the fluid pathway;
a fluid reservoir disposed within the housing and fluidly coupled to the fluid pathway;
a plunger moveably disposed within the fluid reservoir; and
a valve cavity disposed into the housing;
a fluid inlet tube coupled to the fluid input port; and
a fluid outlet tube coupled to the fluid output port,
wherein the disposable fluid output portion is configured to be removably coupled to a reusable control portion of the medical fluid flow device.

15. The fluid flow set of claim 14, wherein the fluid reservoir is pre-filled with a flushing medium.

16. The fluid flow set of claim 14, wherein the valve cavity is configured to receive a fluid flow valve of the reusable control portion and the plunger is configured to couple with an actuator of the reusable control portion when the disposable fluid output portion is coupled to the reusable control portion of the medical fluid flow device.

17. The fluid flow set of claim 14, further comprising a sensor cavity disposed into the housing and configured to receive a sensor member of a sensor assembly of the reusable control portion when the disposable fluid output portion is coupled to the reusable control portion of the medical fluid flow device.

18. The fluid flow set of claim 14, wherein the fluid inlet tube coupled to the fluid input port is configured to pass through an open side of a line cavity disposed in a chassis of the reusable control portion when the disposable fluid output portion is coupled to the reusable control portion of the medical fluid flow device.

19. A method of operating a medical fluid flow device, the method comprising:
coupling a fluid flow set to a reusable control portion of a medical fluid flow device, the fluid flow set comprising:
a disposable fluid output portion comprising a housing, an internal fluid pathway, a fluid input port fluidly coupled to the fluid pathway, a fluid output port fluidly coupled to the fluid pathway, a fluid reservoir disposed within the housing and fluidly coupled to the fluid pathway, a plunger moveably disposed within the fluid reservoir and a valve cavity disposed into the housing;
a fluid inlet tube having a first end coupled to the fluid input port; and
a fluid outlet tube having a first end coupled to the fluid output port;
coupling a second end of the fluid inlet tube to an intravenous pump;
coupling a second end of the fluid outlet tube to a medication delivery port;
entering, via a touchscreen of the reusable control portion, at least one of a pump number and a drug name corresponding to the fluid inlet tube;
pressing an on button on the touchscreen to run an infusion of the drug from the intravenous pump to the medication delivery port;
opening, by a fluid flow controller of the reusable control portion, a valve coupled to the fluid flow controller to fluidly connect the fluid inlet tube with the fluid pathway in the disposable fluid output portion;
pressing an off button on the touchscreen to end the infusion of the drug from the intravenous pump to the medication delivery port;
closing, by the fluid flow controller of the reusable control portion, the valve to close the fluid connection between the fluid inlet tube and the fluid pathway in the disposable fluid output portion;
automatically activating an actuator of the reusable control portion to push the plunger into the fluid reservoir in the disposable fluid output portion; and
forcing, by the plunger, a flushing medium from the reservoir to flush through the fluid pathway and out to the fluid outlet tube.

20. The method of claim 19, wherein the fluid flow set is a first fluid flow set, the method further comprising replacing the first fluid flow set by:
disconnecting the disposable fluid output portion of the first fluid flow set from the reusable control portion of the medical fluid flow device;
disconnecting the fluid inlet tube of the first fluid flow set from the intravenous pump;
disconnecting the fluid outlet tube of the first fluid flow set from the medication delivery port;
connecting a disposable fluid output portion of a second fluid flow set having a reservoir pre-filled with a flushing medium to the reusable control portion of the medical fluid flow device;
connecting a fluid inlet tube of the second fluid flow set to the intravenous pump; and
connecting a fluid outlet tube of the second fluid flow set to the medication delivery port.
